Europäisches Patentamt

European Patent Office

Office européen des brevets

(11) Publication number: **0 226 085**
**A2**

# (12) EUROPEAN PATENT APPLICATION

(21) Application number: **86116402.8**

(22) Date of filing: **26.11.86**

(51) Int. Cl.⁴: **A61M 1/14**

(30) Priority: **19.12.85 SE 8506029**

(43) Date of publication of application:
**24.06.87 Bulletin 87/26**

(84) Designated Contracting States:
**BE CH DE ES FR GB IT LI NL**

(71) Applicant: **GAMBRO AB**
**Post Box 10101**
**S-220 10 Lund(SE)**

(72) Inventor: **William-Olsson, Göran Enar William**
**Södra Vägen 59**
**S-412 54 Göteborg(SE)**
Inventor: **Grebius, Staffan**
**Adelgatan 13**
**S-223 50 Lund(SE)**
Inventor: **Beijbom, Peter**
**Uardavägen 6c**
**S-223 71 Lund(SE)**

(74) Representative: **Boberg, Nils Gunnar Erik**
**Gambro AB Patent Department Box 10101**
**S-220 10 Lund(SE)**

(54) **Cardio pulmonary system intended for oxygenation of a patient's blood.**

(57) A cardiopulmonary system intended for the oxygenation of a patient's blood in connection with an operation on or near the heart with the use of the patient's own lung or lungs for this oxygenation, comprising means for the withdrawal of venous blood from the heart or a major vein connected thereto and for the conduction of this blood via a first chamber to one or to both the lungs and from there, oxygenated, via a second chamber to the heart or a major vein connected thereto.

The system in accordance with the invention comprises as the said means a reservoir (13) with at least two chambers (14 and 15), namely one for venous blood and one for arterial blood.

As a result a system is provided which in addition to oxygenation makes possible also other treatment of the blood, e g warming up and/or filtering.

Fig. 1

## CARDIO PULMONARY SYSTEM INTENDED FOR OXYGENATION OF A PATIENT'S BLOOD

### TECHNICAL FIELD

The present invention relates to a cardio pulmonary system intended for the oxygenation of a patient's blood in connection with an operation on or near the heart with the use of the patient's own lung or lungs for this oxygenation, comprising means for the withdrawal of venous blood from the heart or a major vein connected thereto and for the conduction of this blood via a first chamber to one or to both the lungs and from there, oxygenated, via a second chamber to the heart or another major vein connected thereto.

### BACKGROUND ART

A system of the above mentioned type is described by M H Cass and D N Ross in the article "The evolution of a by-pass technique using the lungs as an oxygenator", Guy's Hospital Reports 1959; 108:237-44.

However, after 1959 this system became more or less forgotten and different mechanical arrangements for the oxygenation of the blood were used instead, the oxygen being added either in form of bubbles directly into the blood, as described, for example, in the US patent 4 374 088, or via a gas-permeable but water-tight membrane, as described, for example, in the US patent 3 612 281. In recent times, though, there has been renewed interest in the older system by Andre Bodnar and Donald Nixon Ross, for example, in their article "Bilateral Cardiac Bypass Without an Oxygenator for Coronary Surgery" in Progress in Artificial Organs 1983.

One difficulty with this system is, however, that no appropriate equipment exists, so that those who attempted to make use of the system were compelled to put together complicated systems comprising conventional cardiotomy reservoirs, blood pumps, filters and other control equipment. It is the object of the present invention, therefore, to make possible the use of the abovementioned system, but with the help of a very simple, yet nevertheless reliable, equipment.

### DISCLOSURE OF INVENTION

The invention thus relates to a cardiopulmonary system intended for the oxygenation of a patient's blood in connection with an operation on or near the heart with the use of the patient's own lung or lungs for this oxygenation, comprising means for the withdrawal of venous blood from the heart or a major vein connected thereto, and for the conduction of this blood via a first chamber to one or to both the lungs and from there, oxygenated, via a second chamber to the heart or another major vein connected thereto.

The pricnipal characteristic of the invention in this case is that the said means comprice a reservoir with at least two chambers, namely one for venous blood and one for arterial blood. In this manner, among other things, a regular supply of blood to the lungs is made possible, even if the withdrawal of this venous blood from the patient were to be slightly irregular. At the same time this two-chamber system makes it easily possible for the blood to be treated, e g warmed up, on its way to and from the lungs.

The reservoir preferably comprises a third chamber intended to serve essentially as a cardiotomy reservoir by receiving drawn-up blood and the like. This drawn-up blood can, preferably after filtration, be mixed with the venous blood so as to be oxygenated jointly with the same.

The two firstnamed chambers are preferably arranged to be in connection with each other as two communicating vessels. As a result substantially the same level is maintained in the two chambers, which further facilitates the regular supply of blood to the lungs. In a preferred embodiment of the subject of the invention, this connection is achieved by a preferably fully open, flexible external duct connected with its ends to the chambers near to, or at, their lowest points. As a result the actual design of the chambers can be made very simple, at the same time as the arrangement of any filters and the like is not prevented. Moreover the duct, if it is constituted of a flexible tube, can easily be closed, if necessary, with the help of a spring clip or the like.

To make possible the warming up of the treated blood, the two first mentioned chambers are provided appropriately each with its heat exchanger. This heat exchanger may be formed in a very simple manner by a single unbroken duct in the form of a preferably horizontal helix-shaped spiral with end connections arranged above the maximum blood level. In this manner there is naturally less danger of the heating medium used leaking out into the blood or vice versa.

The heat exchanger is arranged appropriately at the bottom of the respective chamber, which may be in the shape of a semicylinder. If at the same time its centre core is filled out with a cylinder displacing the blood, a very effective design is obtained which only requires a small amount of blood to function.

Even if the blood is withdrawn substantially direct from the heart or from the lungs the inlets to the two first mentioned chambers are arranged so as to be separated from their respective main parts by a filter. This is even more important for the third chamber, which preferably is adapted so as to be in communication with the chamber intended to receive venous blood via at least one filter. The said filters are built up appropriately in conventional manner from a defoamer material, e g. antifoam-treated polyurethane together with a tricot fabric, e g of nylon or polyester, or a deep filter.

A particularly simple design from a point of view of manufacture is obtained, if the reservoir is built up according to a cassette system with preferably a fixed wall between the two first mentioned chambers and with a number of loosely insertable filters arranged between the different inlets and the respective chambers.

To facilitate the level control between the two first mentioned chambers, without risking any contamination of the same, they are appropriately in connection with the outside atmosphere via a sterile filter provided near to, or at, their highest point.

Beside the above described reservoir the system in accordance with the invention essentially needs only to contain a first duct for the withdrawal of venous blood from the heart or a major vein connected thereto and the conduction of this blood the the chamber for venous blood, a second duct with a pump connected thereto for the pumping of the blood from this chamber to either, or to both, lungs, a third duct for the withdrawal of oxygenated blood from the heart or a major vein connected thereto and the conduction of this blood to the chamber for arterial blood and a fourth duct with a pump connected thereto. It is appropriate, however, for the system to contain a fifth duct with a pump connected thereto which is coupled to a blood suction device or the like.

BRIEF DESCRIPTION OF DRAWINGS

The invention will be described in greater detail in the following with reference to the attached drawings.

Fig 1 illustrates schematically, by way of example, a relatively complete system in accordance with the invention.

Fig 2 shows a reservoir included in the system in accordance with Fig 1 seen from the top.

Fig 3 shows the same reservoir, partly seen in section through a chamber included therein.

Fig 4, finally, shows in form of an exploded view the set-up in principle of a filter included in the said reservoir.

BEST MODE OF CARRYING OUT THE INVENTION

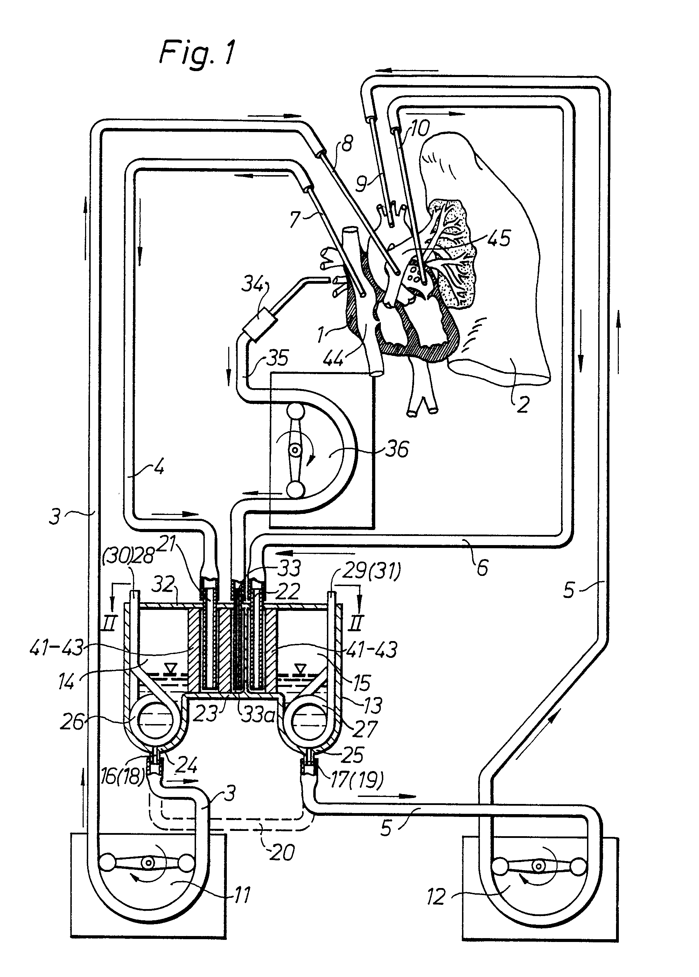

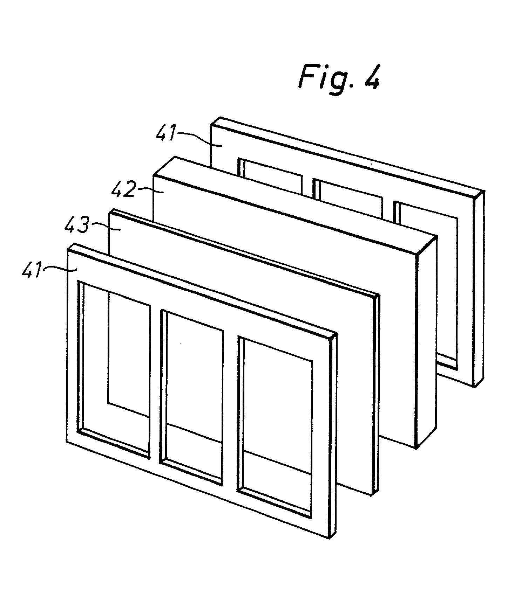

Fig 1, thus, shows an essentially complete system in acccordance with the invention and is intended to illustrate how the system will be coupled to a patient's heart 1 and lungs 2. This is done with the help of ducts 3, 4, 5 and 6 attached to catheters or cannulas 7, 8, 9 and 10. The flow in the ducts 3 and 5 in this case is meant to be achieved with the help of pumps 11 and 12. The flow in the ducts 4 and 6 is achieved by the blood's own pressure, possibly reinforced by the force of gravity, in that a reservoir 13, to which all ducts are connected, is placed lower than the patient. This reservoir 13 comprises in the first place two chambers 14 and 15 intended for venous and arterial blood, respectively. The ducts 3 and 5 lead away from two nipples 16 and 17 arranged at the lowest point of the respective chamber. As is evident from Fig 3, two further nippels 18 and 19 are provided which, as shown schematically in Fig 1 by a broken line, are connected with each other through duct 20, so that the chambers 14 and 15 form two communicating vessels with substantially the same liquid level. The ducts 3 and 5 and the duct 20, respectively, are thus situated in different planes in Fig 1. The ducts 4 and 6 open into two inlet pipes 21 and 22, respectively, which in turn open out near the raised intermediate base 23 of the reservoir 13.

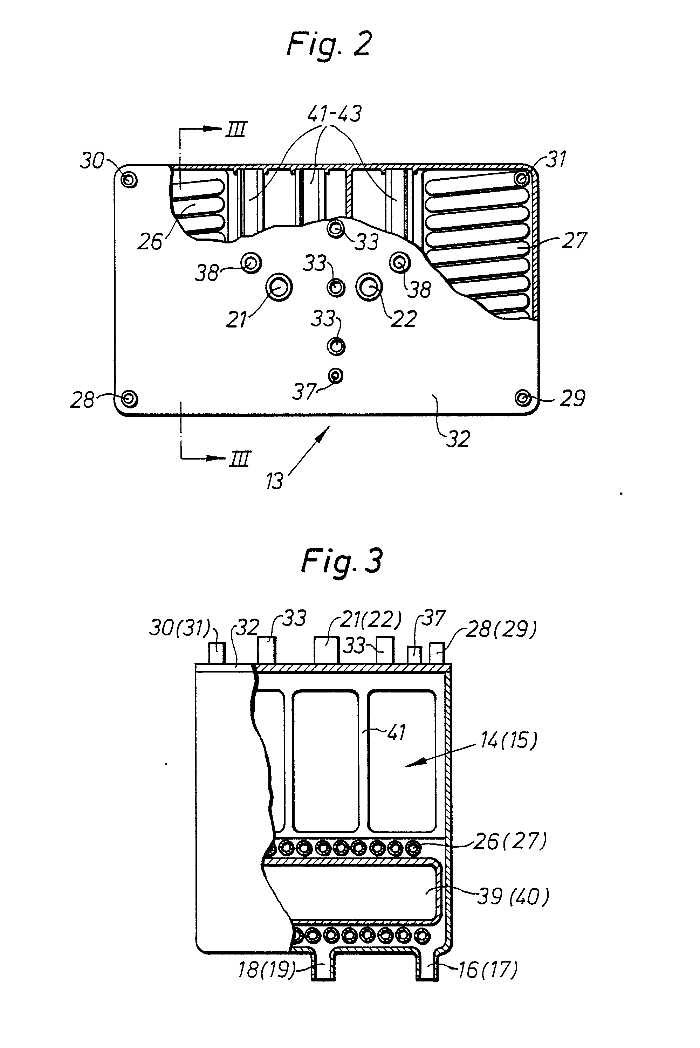

On either side of this intermediate base the chambers 14 and 15, respectively, are designed to have a substantially semicylindrical chamber base 24 and 25, respectively. These chamber bases are designed so as to be adapted to two heat exchangers 26 and 27, each in the form, of a horizontal helix-shaped spiral, whose inlets and outlets 28-31 are arranged outside the respective chamber, that is to say clearly above the maximum blood level in accordance with the definition in the following claims. These inlets and outlets are best visible in fig 2 which shows the reservoir 13 seen from the top with its cover 32 partly removed. The inlet pipes 21 and 22 in this case are shown as two circles. Three further inlet pipes 33 are intended to be connected to one or more suction devices 34, one of which is shown in Fig 1. This connection is done via a duct 35 with a suction pump 36 joined thereto. These inlet pipes 33 open out into a common chamber which is designated 33a. A further inlet pipe 37, not shown in Fig 1, is intended to be used for the filing in of priming fluid, that is to say a physiologically acceptable salt solution, which fills up the system until it is replaced by blood. Two further circles 38 in Fig 2 indicate two gas outlets through which any gas set free can be descharged, which is done appropriately via two sterile filters.

In Fig 3 is shown how the respective heat exchangers 26, 27 are filled out with a cylindrical core 39 and 40, respectively. Fig 3, thus, represents a section through one or the other of the chambers 14 and 15. On the top in Fig 3 are found again the different connections 21, 22, 28-31, 33 and 37, the connections 21 and 22, thus, being arranged straight before each other whilst also consealing one of the connections 33. A frame, which is indicated by designation 41, in a cassettelike pack, is shown in greater detail in Fig 4. This pack may consist of two such frames 41 with a coarse filter 42 and a fine filter 43 arranged in between. The coarse filter 42 may consist, for example, of antifoam-treated polyurethane at the same time as the fine filter 43 may be constituted, for example, of a tricot fabric, e g of nylon or polyester. Two such cassettelike filters are shown schematically in Fig 1 and 2 arranged between the inlet pipes 21 and 22, respectively, and adjoining chambers 14 and 15, respectively. A third similar cassettelike filter may be arranged between the inlet pipe 33 and the inlet pipe 21, that is to say so as to achieve an extra filtration of blood from the suction device 34. In the last named filter the tricot fabric is replaced appropriately by a so called deep filter or a so called screen filter.

INDUSTRIAL APPLICABILITY

Fig 1 is, thus, intended to illustrate the application of the system in accordance with the invention. This application should not require any further explanation for those versed in the art. Summing it up, it takes place in such a manner that venous blood is withdrawn with the help of the catheter 7 from vena cava 44 and is made to flow by its own pressure through the duct 4, the inlet pipe 21 and the filter 41-43 to the chamber 14. From there it is pumped with the help of the pump 11 through the duct 3 and the catheter 8 to the pulmonary artery 45 and, thus, into the lung or lungs 2. From here the blood passes to the left atrium from which it is withdrawn with the help of the catheter 10 and is conducted by means of the duct 6 to the inlet pipe 22 and via a second filter 41-43 to the chamber 15.

After suitable warming in this chamber the blood is passed with the help of the pump 12 on through the duct 5 and the catheter 9 to the aorta or to one or more other major arteries.

Naturally the invention is not limited simply to the embodiment described above, but may be varied within the scope of the following claims. Certain possible variations are evident also from the afore mentioned articles, whose content, therefore, is included in the present description.

Claims

1. A cardiopulmonary system intended for the oxygenation of a patient's blood in connection with an operation on or near the heart with the use of the patient's own lung or lungs for this oxygenation, comprising means for the withdrawal of venous blood from the heart or a major vein connected thereto and for the conduction of this blood via a first chamber to one or to both the lungs and from there, oxygenated, via a second chamber to the heart of a major vein connected thereto, **characterized** in that the said means comprise a reservoir (13) with at least two chambers (14, 15) namely one for venous blood and one for arterial blood.

2. A system in accordance with claim 1, **characterized** in that the reservoir (13) comprises a third chamber (33) intended to serve essentially as a cardiotomy reservoir by receiving drawn-up blood and the like.

3. A system in accordance with claim 1 or 2, **characterized** in that the two firstnamed chambers (14, 15) are arranged to be in connection with each other as two communicating vessels.

4. A system in accordance with claim 3, **characterized** in that the two chambers (14, 15) are in connection with each other through a preferably fully open, flexible duct (20) connected with its ends to the chambers near to, or at, their lowest points.

5. A system in accordance with anyone of the preceding claims, **characterized** in that the two first mentioned chambers (14, 15) are provided preferably each with its heat exchanger (26, 27).

6. A system in accordance with claim 5, **characterized** in that the heat exchanger or the heat exchangers (26, 27) are constituted of an unbroken duct in the form of a preferably horizontal helix-shaped spiral with end connections (28-31) arranged above the maximum blood level.

7. A system in accordance with claim 6, **characterized** in that the heat exchanger (26, 27) is arranged at the bottom of the chamber which is in the shape of a semicylinder, and that its centre core is filled out with a cylinder (39, 40) displacing the blood.

8. A system in accordance with anyone of the preceding claims, **characterized** in that the inlets (21, 22) to the first mentioned two chambers (14, 15) are separated from their main part by a filter - (41-43).

9. A system in accordance with anyone of the preceding claims, **characterized** in that the said third chamber (33a) is in communication with the chamber (14), which is intended to receive venous blood, via at least one filter (41, 43).

10. A system in accordance with anyone of the preceding claims, **characterized** in that the reservoir (13) is built up according to a cassette system with preferably a fixed wall between the two first mentioned chambers (14, 15) and with a number of loosely insertable filters (41-43) arranged between the different inlets and the respective chambers.

11. A system in accordance with anyone of the preceding claims, **characterized** in that at least the two first mentioned chambers (14, 15) are in connection with the outside atmosphere via a sterile filter (38) provided near to, or at, their highest point.

12. A system in accordance with anyone of the preceding claims, **characterized** by a first duct for the withdrawal of venous blood from the heart (1) or a major vein connected thereto and the conduction of this blood to the chamber for venous blood and a second duct (3) with a pump (11) connected thereto for the pumping of the blood from this chamber (14) to either, or to both, lungs (2).

13. A system in accordance with anyone of the preceding claims, **characterized** by a third duct - (6) for the withdrawal of oxygenated blood from the heart (1) or a major vein connected thereto and the conduction of this blood to the chamber (15) for arterial blood and a fourth duct (5) with a pump - (12) connected thereto for the pumping of the blood from this chamber (15) to the heart (1) or a major artery connected thereto.

14. A system in accordance with anyone of claims 8-10, **characterized** in that the said filter is built up of a defoamer material, e g antifoam-treated polyurethane together with a tricot fabric, e g of nylon or polyester, or a deep filter.

*Fig.1*

Fig. 2

Fig. 3

## Fig. 4